# EUROPEAN PATENT APPLICATION

(11) **EP 1 400 520 A1**
(43) Date of publication of application: **24.03.2004**
(21) Application number: 02720592.1
(22) Date of filing: 25.04.2002
(51) Int. Cl.: C07D 401/12, A61K 31/501, A61P 9/12, A61P 43/00

(54) **CRYSTAL OF 6- 4-(4-PYRIDYLAMINO)PHENYL]-4, 5-DIHYDRO-3(2H)-PYRIDAZINONE HYDROCHLORIDE TRIHYDRATE**

(30) Priority: 27.04.2001 JP 2001130767
(71) Applicant: Mitsubishi Pharma Corporation, Osaka-shi, Osaka 541-0046 (JP)
(72) Inventor: ASATANI, Haruki, Mitsubishi Chemical Corporation, Yokohama-shi, Kanagawa 227-8502 (JP); TSUBOI, Akio, Mitsubishi Chemical Corporation, Kurashiki, Okayama 712-8054 (JP); ZHANG, Suojiang, PC. 100080 Beijing (CN); OHURA, Shinji, MITSUBISHI PHARMA CORPORATION, Chuo-ku, Tokyo 103-8405 (JP)
(74) Representative: Sternagel, Fleischer, Godemeyer & Partner Patentanwälte
(86) International application number: PCT/JP2002/004121
(87) International publication number: WO 2002/088109

(57) **Abstract**

The present invention provides crystals of 6-[4-(4-pyridylamino)phenyl]-4,5-dihydro-3(2H)-pyridazinone hydrochloride trihydrate useful as a medicament for therapeutic treatment of cardiac failure, and the like; and a pharmaceutical composition comprising said crystals as an active ingredient.

The crystal of the present invention has characteristic absorption peaks (2 θ degrees) at 12.9 (±0.2°) and 19.0 (±0.2°), for example, in powder X-ray diffractometry.

## Description

### Field of the Invention

The present invention relates to crystals of 6-[4-(4-pyridylamino)phenyl]-4,5-dihydro-3(2H)-pyridazinone hydrochloride trihydrate useful as a medicament for therapeutic treatment of cardiac failure and the like.

### Background of the Invention

Japanese Patent Unexamined Publication (KOKAI) No. (Sho) 61-289032/1986 discloses various pyridazinone derivative compounds which have cardiac action. Among them, 6-[4-(4-pyridylamino)phenyl]-4,5-dihydro-3(2H)-pyridazinone hydrochloride represented by the following formula (I) 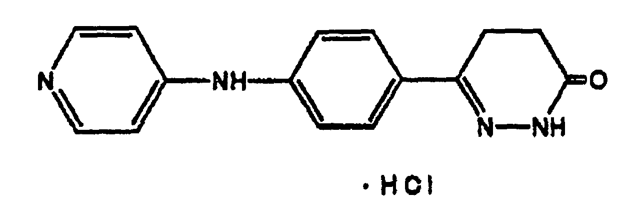 is known to have superior cardiac action and to be a useful compound as a medicament. However, Japanese Patent Unexamined Publication (KOKAI) No. (Sho) 61-289032/1986 only discloses a method for preparation of 6-[4-(4-pyridylamino)phenyl]-4,5-dihydro-3(2H)-pyridazinone hydrochloride. Although the chemical structure, per se, of a trihydrate of said hydrochloride is known, no method for preparation of the trihydrate of said hydrochloride is specifically known. Therefore, as for crystals of 6-[4-(4-pyridylamino)phenyl]-4,5-dihydro-3(2H)-pyridazinone hydrochloride trihydrate, no information is available as to what kind of crystalline forms may possibly exist, as well as how they can be prepared.

When a compound in a crystalline form is used as a medicament, it is important that the crystals are stably supplied and have a constant quality. However, as for crystals of 6-[4-(4-pyridylamino)phenyl]-4,5-dihydro-3(2H)-pyridazinone hydrochloride trihydrate, no information is available as to what type of crystalline form can be stably supplied.

An object of the present invention is to provide, among 6-[4-(4-pyridylamino)phenyl]-4,5-dihydro-3(2H)-pyridazinone hydrochlorides represented by formula (I), a trihydrate in a crystalline form. A further object is to provide a crystalline form obtained by a preparation method that enables stable supply of the crystals when they are used as a medicament.

### Disclosure of the Invention

The inventors of the present invention conducted intensive researches on 6-[4-(4-pyridylamino)phenyl]-4,5-dihydro-3(2H)-pyridazinone hydrochloride to achieve the foregoing objects, and as a result, they succeeded in obtaining two types of crystals as being trihydrates. They also found that one of these crystals was obtainable by a method that enables stable supply of the crystals, and that the crystal was useful as a medicament. They thus achieved the present inventions.

The gists of the present invention are as follows:
(1) A crystal of 6-[4-(4-pyridylamino)phenyl]-4,5-dihydro-3(2H)-pyridazinone hydrochloride trihydrate.
(2) The crystal according to (1), characterized to have characteristic absorption peaks (cm⁻¹) at 1354, 1523-1524, 1648-1644, 2946-2947, 3217-3218, and 3479-3484 by infrared absorption measurement (IR).
(3) The crystal according to (1) or (2), characterized to have characteristic absorption peaks (2 θ degrees) at 12.9 (±0.2°) and 19.0 (±0.2°) by powder X-ray diffractometry (XRD).
(4) The crystal according to (1) or (2), characterized to have characteristic absorption peaks (2 θ degrees) at 11.8 (±0.2°), 12.4 (±0.2°), 12.9 (±0.2°), 17.9 (±0.2°), and 19.0 (±0.2°) by XRD.
(5) The crystal according to (1) or (2), characterized to have characteristic absorption peaks (2 θ degrees) at 12.9 (±0.2°), 19.0 (±0.2°), 21.0 (±0.2°), 24.2 (±0.2°), 24.5 (±0.2°), 24.9 (±0.2°), and 28.7 (±0.2°) by XRD.
(6) The crystal according to (1) or (2), characterized to have characteristic absorption peaks (2 θ degrees) at 11.3 (±0.2°), 12.4 (±0.2°), 12.9 (±0.2°), 17.9 (±0.2°), 19.0 (±0.2°), 21.0 (±0.2°), 22.8 (±0.2°), 24.2 (±0.2°), 24.5 (±0.2°), 24.9 (±0.2°), 28.7 (± 0.2°), and 29.1 (±0.2°) by XRD.
(7) The crystal according to any one of (1) to (7), characterized to have an endothermic peak at around 77°C, and have about 14.6 % weight decrease ratio at around 67°C by thermogravimetry and differential thermal analysis (TG-DTA).
(8) The crystal according to any one of (1) to (8), characterized not to have an endothermic peak at around 120°C to 140°C by differential scanning calorimetry (DSC).
(9) The crystal according to (1) or (2) characterized to have a characteristic absorption peak (2 **θ** degrees) at 12.4 (±0.2°) by XRD.
(10) The crystal according to (1) or (2), characterized to have characteristic absorption peaks (2 θ degrees) at 11.1 (±0.2°), 11.3 (±0.2°), 12.2 (±0.2°), 12.4 (±0.2°), and 17.9 (±0.2°) by XRD.
(11) The crystal according to (1) or (2), characterized to have characteristic absorption peaks (2 θ degrees) at 12.4 (±0.2°), 20.5 (±0.2°), 21.6 (±0.2°), 24.9 (±0.2°), 80.2 (±0.2°), 80.9 (±0.2°), 33.3 (±0.2°), and 87.8 (±0.2°) by XRD.
(12) The crystal according to (1) or (2), characterized to have characteristic absorption peaks (2 θ degrees) at 11.1 (±0.2°), 11.3 (±0.2°), 12.2 (±0.2°), 12.4 (±0.2°), 17.9 (±0.2°), 20.5 (±0.2°), 21.6 (±0.2°), 22.6 (±0.2°), 24.9 (±0.2°), 29.1 (±0.2°), 30.2 (± 0.2°), 30.9 (±0.2°), 33.3 (±0.2°), and 37.8 (±0.2°) by XRD.
(18) The crystal according to any one of (1), (2), and (10) to (12), characterized to have endothermic peaks at around 80°C, 148°C, and 150°C; have about 13.8 % weight decrease ratio at around 70°C; and have about 1.6 % weight decrease ratio at around 141°C by TG-DTA.
(14) The crystal according to any one of (1), (2), and (10) to (13), characterized to have an endothermic peak at around 120°C to 140°C by DSC.
(16) A pharmaceutical formulation which comprises, as an active ingredient, the crystal of 6-[4-(4-pyridylamino)phenyl]-4,5-dihydro-3(2H)-pyridazinone hydrochloride trihydrate according to any one of (1) to (14), and a pharmaceutically acceptable carrier.
(16) A medicament for cardiac failure which comprises, as an active ingredient, the crystal of 6-[4-(4-pyridylamino)phenyl]-4,5-dihydro-3(2H)-pyridazinone hydrochloride trihydrate according to any one of (1) to (14), and a pharmaceutically acceptable carrier.
(17) An antihypertensive agent which comprises, as an active ingredient, the crystal of 6-[4-(4-pyridylamino)phenyl]-4,5-dihydro-3(2H)-pyridazinone hydrochloride trihydrate according to any one of (1) to (14), and a pharmaceutically acceptable carrier.
(18) An agent for enhancing calcium ion sensitivity which comprises, as an active ingredient, a crystal of 6-[4-(4-pyridylamino)phenyl]-4,5-dihydro-3(2H)-pyridazinone hydrochloride trihydrate according to any one of (1) to (14).
(19) A cardiant which comprises, as an active ingredient, the crystal of 6-[4-(4-pyridylamino)phenyl]-4,5-dihydro-3(2H)-pyridazinone hydrochloride trihydrate according to any one of (1) to (14).
(20) An ophthalmologic agent which comprises, as an active ingredient, the crystal of 6-[4-(4-pyridylamino]phenyl]-4,5-dihydro-3(2H)-pyridazinone hydrochloride trihydrate according to any one of (1) to (14).

### Brief Explanation of the Drawings

Figure 1 shows powder X-ray diffraction patterns of the type B crystal and the type B' crystal.
Figure 2 shows a TG-DTA curve of the type B crystal.
Figure 3 shows a TG-DTA curve of the type B' crystal.
Figure 4 shows an IR pattern of the type B crystal.
Figure 5 shows an IR pattern of the type B' crystal.
Figure 6 shows a DSC curve of the type B crystal.
Figure 7 shows a DSC curve of the type B' crystal.

### Beat Mode for Carrying out the Invention

The present invention is described in further detail below.

The crystal of 6-[4-(4-pyridylamino)phenyl]-4,5-dihydro-3(2H)-pyridazinone hydrochloride trihydrate of the present invention is represented by the following chemical formula.

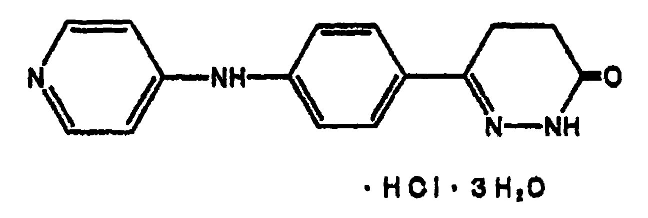

The crystal of the present invention has characteristic absorption peaks (cm⁻¹) at 1354, 1523-1524, 1643-1644, 2946-2947, 3217-3218 and 3479-3484 by IR measurement.

Crystals provided by the present invention are two types of crystals, i.e., Type B crystal and Type B' crystal. Characteristics of each crystal are described below. Type B crystal:

In powder X-ray diffractometry, the crystal is characterized to have characteristic absorption peaks (2 θ degrees) at 12.9 (±0.2°) and 19.0 (±0.2°), preferably to have characteristic absorption peaks (2 θ degrees) at 11.3 (±0.2°), 12.4 (±0.2°), 12.9 (±0.2°), 17.9 (±0.2°), and 19.0 (±0.2°), more preferably to have characteristic absorption peaks (2 θ degrees) at 12.9 (±0.2°), 19.0 (±0.2°), 21.0 (±0.2°), 24.2 (±0.2°), 24.5 (±0.2°), 24.9 (±0.2°), and 28.7 (±0.2°), most preferably to have characteristic absorption peaks (2 θ degrees) at 11.8 (±0.2°), 12.4 (±0.2°), 12.9 (±0.2°), 17.9 (±0.2°), 19.0 (±0.2°), 21.0 (±0.2°), 22.8 (±0.2°), 24.2 (±0.2°), 24.5 (±0.2°), 24.9 (±0.2°), 28.7 (± 0.2°), and 29.1 (±0.2°).

In TG-DTA, the crystal is characterized to have an endothermic peak at around 77°C, and have about 14.5 % weight decrease ratio at around 67°C.

In DSC, the crystal is characterized not to have an endothermic peak at around 120°C to 140°C.

### Type B' crystal:

In powder X-ray diffractometry, the crystal is characterized to have characteristic absorption peaks (2 θ degrees) at 12.4 (±0.2°), preferably to have characteristic absorption peaks (2 θ degrees) at 11.1 (±0.2°), 11.3 (±0.2°), 12.2 (±0.2°), 12.4 (±0.2°), and 17.9 (±0.2°), more preferably to have characteristic absorption peaks (2 θ degrees) at 12.4 (±0.2°), 20.5 (±0.2°), 21.6 (±0.2°), 24.9 (±0.2°), 30.2 (±0.2°), 30.9 (±0.2°), 33.3 (±0.2°), and 37.8 (±0.2°), particularly preferably to have characteristic absorption peaks (2 θ degrees) at 11.1 (±0.2°), 11.3 (±0.2°), 12.2 (±0.2°), 12.4 (±0.2°), 17.9 (±0.2°), 20.5 (±0.2°), 21.6 (±0.2°), 22.6 (±0.2°), 24.9 (±0.2°), 29.1 (±0.2°), 30.2 (± 0.2°), 30.9 (±0.2°), 33.3 (±0.2°), and 37.8 (±0.2°) in powder X-ray diffractometry.

In TG-DTA, the crystal is characterized to have endothermic peaks at around 80°C, 143°C, and 150°C; have about 13.3 % weight decrease ratio at around 70°C; and have about 1.6 % weight decrease ratio at around 141°C.

In DSC, the crystal is characterized to have an endothermic peak at around 120°C to 140°C.

The crystals of the present invention can be prepared by the methods described below.

Methods for preparation of 6-[4-(4-pyridylamino)phenyl]-4,5-dihydro-3(2H)-pyridazinone hydrochloride represented by the above formula (I) are not particularly limited. For example, the methods described in the Japanese Patent Unexamined Publication No. (Sho) 59-186946/1984, Journal of Medicinal Chemistry, 17, 273-181, 1974, the Japanese Patent Unexamined Publication No. (Sho) 60-126282/1985 and the like may be used in combination.

The hydrochloride obtained above is added to a suitable solvent and dissolved. Then a pH of the resulting solution is adjusted with an alkali to obtain crystals of the molecular form of the compound. The resulting crystals are dissolved in a suitable solvent and purified by using active charcoal and silica gel. Hydrochloric acid is then added to the purified solution to convert the molecular compound to a monohydrochloride. The monohydrochloride is crystallized from the solution by cooling or the like. After trituration, water is added to the resulting crystals to obtain a trihydrate. The crystals so obtained are Type B crystals of 6-[4-(4-pyridylamino)phenyl]-4,5-dihydro-3(2H)- pyridazinone hydrochloride trihydrate.

Type B' crystals can be obtained by subjecting the above obtained Type B crystals to a high pressure treatment at a relatively high temperature, at about 65°C, as described in the examples below.

As described above, the two types of crystals, a type B crystal and a type B' crystal, are obtained as crystals of 6-[4-(4-pyridylamino)phenyl]-4,5-dihydro-3(2H)-pyridazinone hydrochloride trihydrate. In the present invention, Type B crystal is preferable which is obtained by a preparation method which enables more stable supply of the crystals.

The crystals of 6-[4-(4-pyridylamino)phenyl]-4,5-dihydro-3(2H)-pyridazinone hydrochloride trihydrate obtained above can be used, per se, or as a pharmaceutical composition prepared by formulation with a carrier acceptable as a medicament.

When the active ingredient of the medicament of the present invention is clinically applied, it is preferable to administer 0.001-1 mg/kg of the aforementioned compound once to three times a day for oral administration. It is preferable to administer 0.001-2 mg/kg of the aforementioned compound twice to five times a day for intravenous administration or the above doses are injected continuously as drip infusion. Further, it is preferable to administer 0.001-1 mg/kg of the aforementioned compound once to three times a day for intrarectal administration. These doses described above are appropriately increased or decreased for application depending on the age, pathological conditions, sexuality, symptoms and the like of a patient.

For formulation of the medicament, the crystals of 6-[4-(4-pyridylamino)phenyl]-4,5-dihydro-3(2H)-pyridazinone hydrochloride trihydrate can be used as a composition comprising a pharmaceutical carrier ordinarily used, i.e., excipients or other additives. The carrier may be solid or liquid.

When a solid carrier is used, the pharmaceutical composition may be in forms of tablets, powders, granules, hard gelatin capsules, suppositories, troches or the like. Any amount of the solid carrier may be used, and preferably an amount of about 1mg to about 1 g may be chosen.

When a liquid carrier is used, the composition may be formulated as syrups, emulsions, soft gelatin capsules, sterilized injections which are typically filled in ampules, and aqueous or non-aqueous suspensions.

The pharmaceutical composition of the present invention can be used as a medicament for cardiac failure, an antihypertensive agent, an agent for enhancing sensitivity for calcium ions, a cardiant, an ophthalmologic agent and the like.

### Examples

The present invention will be specifically explained by referring to examples. However, the scope of the present invention is not limited to these examples.

### Example 1

### Preparation of Type B crystals

6-[4-(4-Pyridylamino)phenyl]-4,5-dihydro-3(2H)-pyridazinone hydrochloride (85 g), prepared by the method similar to that described in example 1 of the Japanese Patent Unexamined Publication No. (Sho) 60-128282/1985, was added to a solvent consisting of about 1050 ml of acetone and 1060 ml of water, and dissolved in the solvent. The solution was neutralized with an aqueous NaOH solution to convert the hydrochloride to a molecular form. After aging, the mixture was filtered, and the solids were washed with an acetone-water mixture (about 390 ml of acetone and about 310 ml of water) and dried to obtain crystals. The resulting crystals (66 g) were added to a heated solvent consisting of about 800 ml of DMF and about 530 ml of water and completely dissolved. Then the solution was cooled and aged, and the crystals were collected by filtration and washed with acetone, and dried to obtain crystals. The crystals obtained (60 g) were added to a solvent consisting of about 2800 g of ethanol and about 180 ml of water, and dissolved. The solution was treated with active charcoal (12 g of active charcoal), and purified by passing through a silica gel column (about 120 g of silica gel). After the solution was filtered with 0.2 µm filter, the water content in the solution was adjusted to 0.6 % by azeotropic dehydration using ethanol. A saturated solution of hydrochloric acid gas in ethanol was then dropped into the dehydrated solution. After the solution was cooled, the crystals were collected by filtration, washed with acetone, and dried to obtain crystals. The resulting crystals (59 g) were triturated, and about 11 g of water was added to adjust the water content in the crystals to 14.5% to 16.5 % (theoretical value: 15.1%). The hydrated crystals were triturated to obtain Type B crystal.

### Example 2

### Preparation of Type B' crystals

Type B crystals (a maximum soluble amount at saturation in water + excess 10 g) obtained in the example 1 were filled with about 10 ml of water into a high pressure container of a small-sized test apparatus for high pressure crystallization (a small-sized test apparatus for high pressure crystallization manufactured by Kobe Steel Ltd., maximum pressure: 400 MPa, temperature range: -20 to 160°C). Pressure was applied stepwise by 50 MPa increments up to the maximum 380 MPa at a constant temperature 75 °C; and the pressure was maintained for 4.5 hours. After the pressure was applied for 4.5 hours, the sample was recovered from the container. Water was removed by a rapid suction filtration (30 minutes) and crystals were dried in air to obtain Type B' crystals. The vacuum pump used for the suction filtration was DA60D manufactured by SINKU KIKO (maximum capacity: 72 L/minute, maximum pressure: 3.32 KPa, maximum vacuum: 24.9 Torr). As a filter paper, 1.0 µm membrane filter (material: esters mixed with cellulose) manufactured by ADVANTEC was used.

### Example 8

### Powder X-ray diffraction, TG-DTA, and water content measurement by Karl Fischer titration

Each of the crystals obtained in the above Example 1 and Example 2 was subjected to a powder X-ray diffraction measurement, thermogravimetry and differential thermal analysis (TG-DTA), and a measurement of water content by Karl Fischer titration. The powder X-ray diffraction and TG-DTA measurements were conducted under the following conditions.

### Powder X-ray diffraction

Apparatus: RINT-1500 manufactured by RIGAKU DENKI
Condition: (θ/2 θ scanning) measurement
   Target: Cu
   Monochromation: By monochromator
   Target output: 40 kV-200 mA
   Monochromator slit for receiving lights: 0.6 mm
   Slit: divergence 1/2°, scattering 1/2°, receiving lights 0.15 mm
   Sampling width: 0.02°
   Integration time: 1 second
   Measured range (2 θ): 5° to 50°

### Thermogravimetry and Differential thermal analysis (TG-DTA)

Apparatus: RIGAKU TG-DTA 220 manufactured by RIGAKU DENKI
   Heating pattern: Room temperature to 200°C; Programmed rate at 10°C/minute
   Atmosphere: Inert gas (N2, 200 ml/ minute)
   Reference: Powdered alumina
   Sample container: Alumina
   Sample weight: about 10 mg

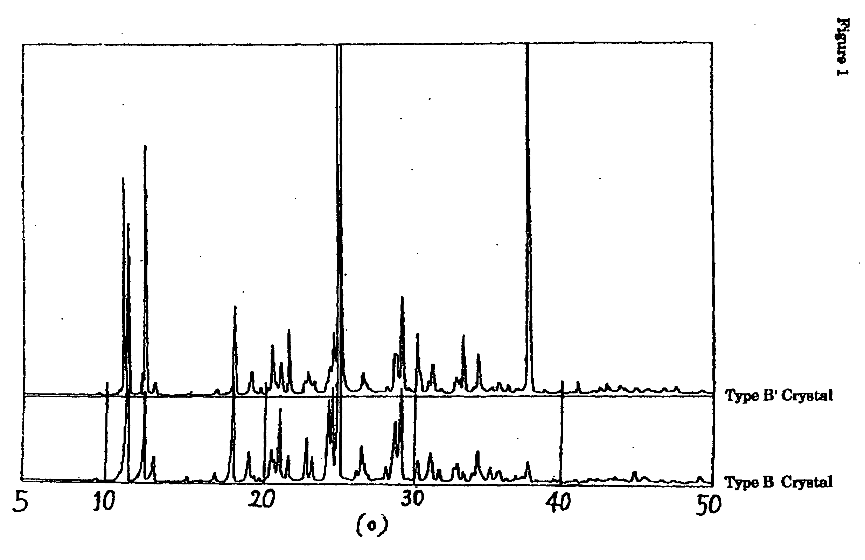

The results of powder X-ray diffraction are shown in Figure 1 where the upper figure shows a powder X-ray diffraction pattern of Type B' crystal, and the lower figure shows a powder X-ray diffraction pattern of Type B crystal. Diffraction angles (2 θ) of main peaks of each pattern are given below.

The type B crystal (2 θ): 11.3 (±0.2°), 12.4 (±0.2°), 12.9 (±0.2°), 17.9 (±0.2°), 19.0 (±0.2°), 21.0 (±0.2°), 22.8 (±0.2°), 24.2 (±0.2°), 24.5 (±0.2°), 24.9 (±0.2°), 28.7 (± 0.2°), and 29.1 (±0.2°)

The type B' crystal (2 θ): 11.1 (±0.2°), 11.3 (±0.2°), 12.2 (±0.2°), 12.4 (±0.2°), 17.9 (±0.2°), 20.5 (±0.2°), 21.6 (±0.2°), 22.6 (±0.2°), 24.9 (±0.2°), 29.1 (±0.2°), 30.2 (± 0.2°), 30.9 (±0.2°), 33.3 (±0.2°), and 37.8 (±0.2°)

It is understood that the two crystals are distinguishable as they have different characteristic peaks.

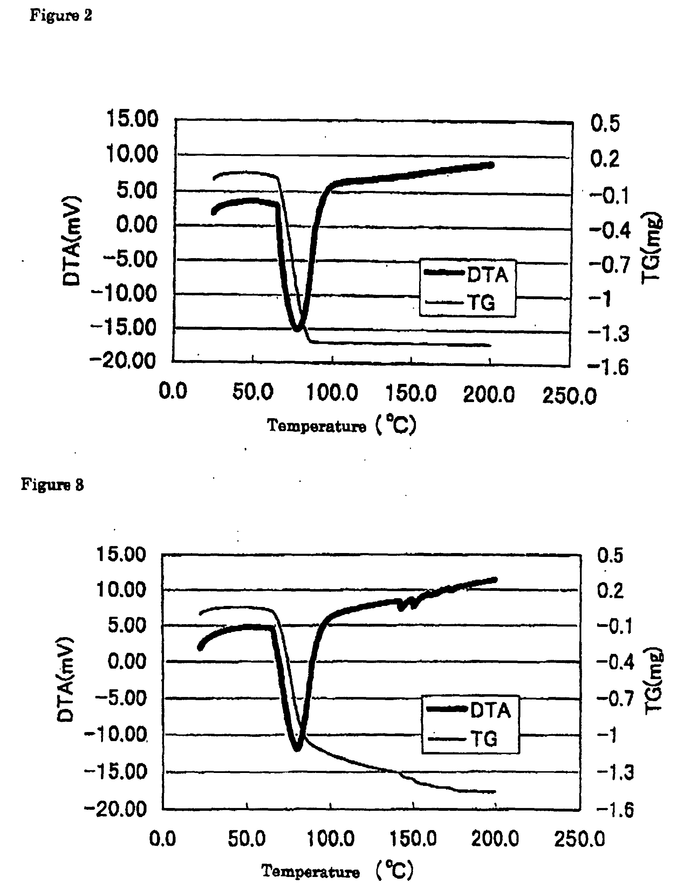

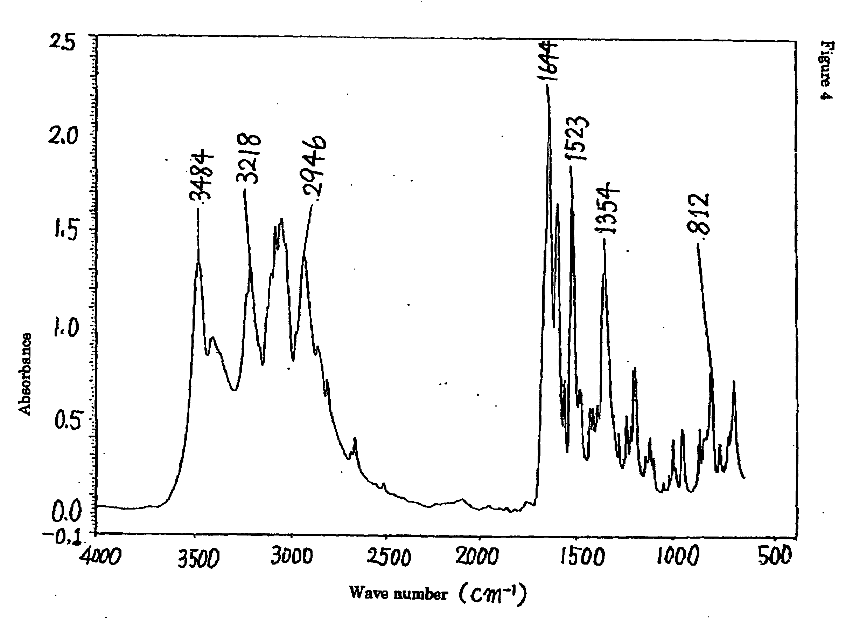

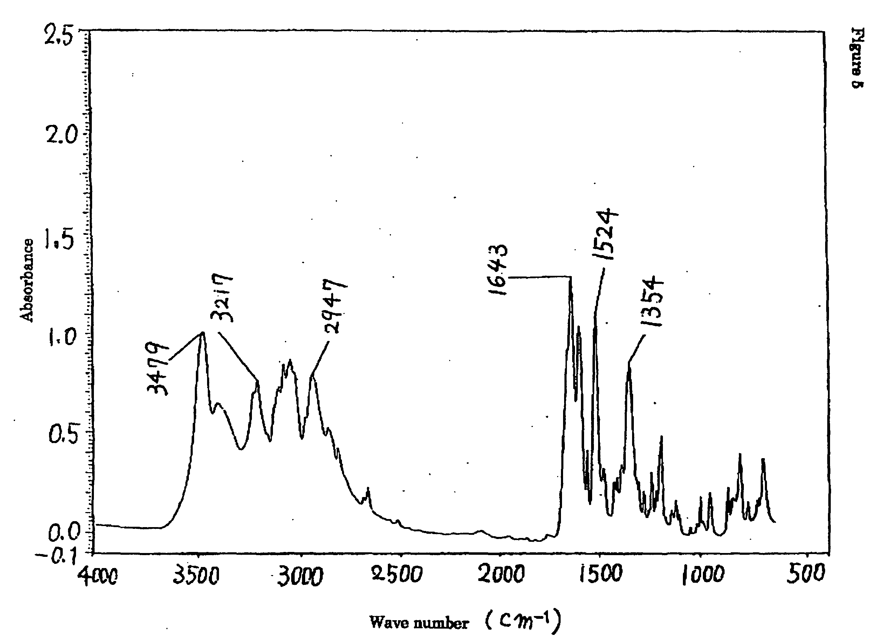

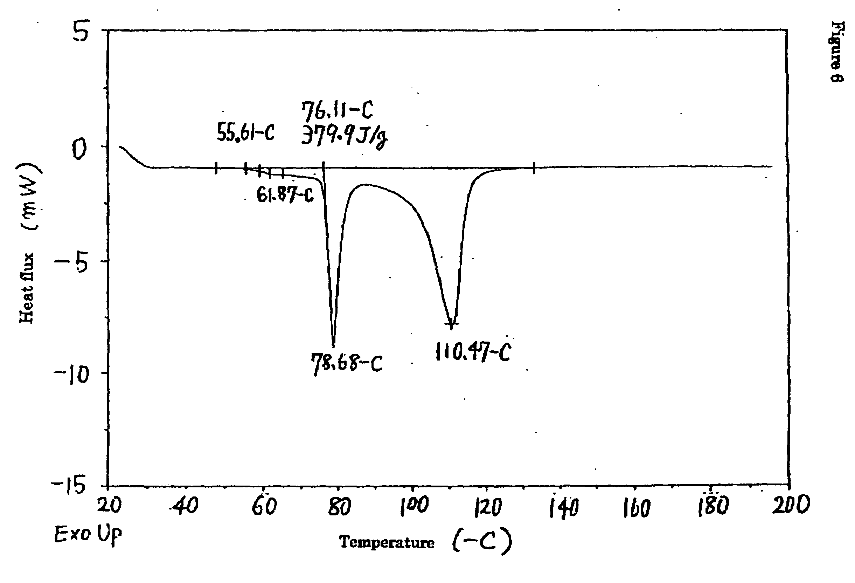

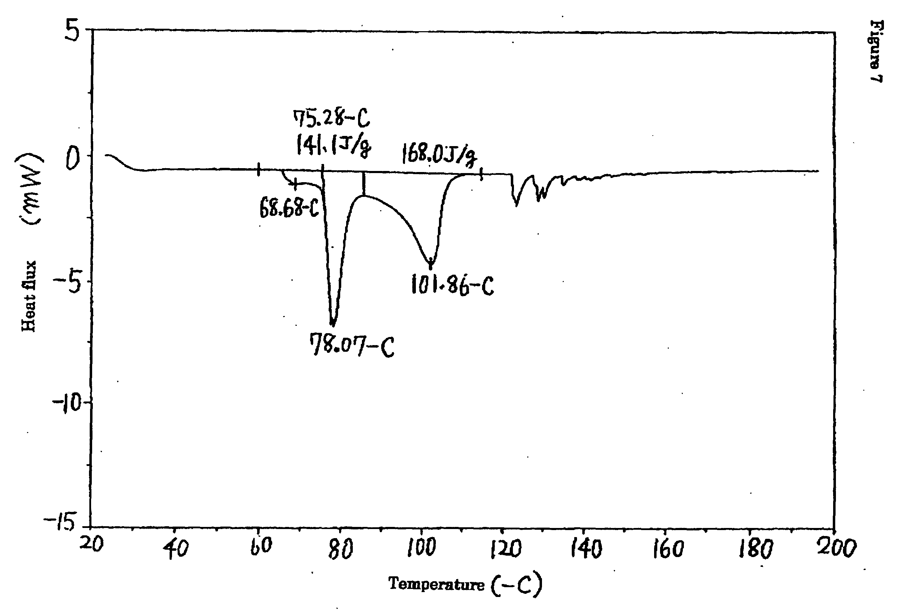

The measured TG-DTA curves are shown in Figure 2 (Type B crystal) and Figure 8 (Type B' crystal). Endothermic peaks and the results of TG are shown in Table 1. From the differences in the endothermic peaks and the TG, it is understood that the two crystals are distinguishable.

**Table 1.**

| Summary of TG-DTA results | | | | |
|---|---|---|---|---|
| Sample | DTA | TG | | |
| | Endothermic peaks (°C) | Property | Break point (°C), Weight loss (%) | |
| Type B crystal | 77 | Break point (°C) | 67 | |
| | | Weight loss (%) | 14.5 | |
| Type B' crystal | 80, 143, 150 | Break point (°C) | 70 | 141 |
| | | Weight loss (%) | 13.8 | 1.6 |

Water content values obtained by Karl Fischer analysis were 14.8 % for Type B crystal, and 15.4 % for Type B' crystal, which clearly indicates that the both crystals are trihydrates.

### Example 3

### Infrared absorbtion and differential scanning calorimetry

Each of the crystals obtained in the above Example 1 and 2 was subjected to infrared (IR) absorption measurement and differential scanning calorimetry (DSC). The IR and DSC measurements were conducted under the following conditions.

### IR measurement

Apparatus: Nicolet-Magna, IR-750 (manufactured by Nicolet)
IR microscopic component; Nic-Plan, IR Microscope (manufactured by Nicolet)
Method: Microscopic FT-IR method (transmission measurement), resolution: 4 cm⁻¹, integration: 64 times, detector: MCT/A. The crystals were leveled up to a thin layer in the diamond cell.

The obtained IR patterns are shown in Figure 4 (Type B crystal) and Figure 6 (Type B' crystal). Differences in the IR spectra of the type B crystal and the type B' crystal were not observed, which indicates that the structures of 6-[4-(4-pyridylamino) phenyl]-4,5-dihydro-3(2H)-pyridazinone hydrochloride trihydrate are maintained. The main wave numbers (peaks) are compared in Table 2.

**Table 2.**

| Main wave numbers (peaks) in IR spectra | |
|---|---|
| Wave number (cm⁻¹) | |
| The type B crystal | the type B' crystal |
| 1354 | 1354 |
| 1528 | 1524 |
| 1644 | 1643 |
| 2946 | 2947 |
| 3218 | 3217 |
| 3484 | 3479 |

### Differential thermal analysis (DSC)

Apparatus: TA-2920 type DSC (manufactured by TA Instrument)
Method: Pin hole method (with 0.32 mm diameter wire)
Temperature range: Room temperature to 200°C, programmed rate: 10°C/minute
Sample container: Standard aluminium sealed container
Sealing atmosphere: Nitrogen

The measured DSC curves are shown in Figure 6 (Type B crystal) and Figure 7 (Type B' crystal). Type B' crystal has a characteristic endothermic peak at 120 to 140°C indicating that the two are different crystals.

### Industrial Applicability

Novel crystals of the compound which is useful as a medicament for cardiac failure can be provided by the present invention. In particular, Type B crystal is excellent from a viewpoint of stable supply, and is useful as a medicament.

The present application was filed with claiming the priority based on the Japanese Patent Application No. 2001-130767. All of the publications in the open literature and documents cited in the present specification, each independently or in a combination, are used as references to the present invention.

## Claims

1. A crystal of 6-[4-(4-pyridylamino)phenyl]-4,5-dihydro-3(2H)-pyridazinone hydrochloride trihydrate.

2. The crystal according to (1), characterized to have characteristic absorption peaks (cm⁻¹) at 1354, 1523-1524, 1643-1644, 2946-2947, 3217-3218, and 3479-3484 by infrared absorption measurement (IR).

3. The crystal according to (1) or (2), characterized to have characteristic absorption peaks (2 θ degrees) at 12.9 (±0.2°) and 19.0 (±0.2°) by powder X-ray diffractometry (XRD).

4. The crystal according to (1) or (2), characterized to have characteristic absorption peaks (2 θ degrees) at 11.3 (±0.2°), 12.4 (±0.2°), 12.9 (±0.2°), 17.9 (±0.2°), and 19.0 (±0.2°) by XRD.

5. The crystal according to (1) or (2), characterized to have characteristic absorption peaks (2 θ degrees) at 12.9 (±0.2°), 19.0 (±0.2°), 21.0 (±0.2°), 24.2 (±0.2°), 24.5 (±0.2°), 24.9 (±0.2°), and 28.7 (±0.2°) by XRD.

6. The crystal according to (1) or (2), characterized to have characteristic absorption peaks (2 θ degrees) at 11.3 (±0.2°), 12.4 (±0.2°), 12.9 (±0.2°), 17.9 (±0.2°), 19.0 (±0.2°), 21.0 (±0.2°), 22.8 (±0.2°), 24.2 (±0.2°), 24.5 (±0.2°), 24.9 (±0.2°), 28.7 (± 0.2°), and 29.1 (±0.2°) by XRD.

7. The crystal according to any one of (1) to (7), characterized to have an endothermic peak at around 77°C, and have about 14.5 % weight decrease ratio at around 67°C by thermal gravimetry differential thermal analysis (TG-DTA).

8. The crystal according to any one of (1) to (8), characterized not to have an endothermic peak at around 120°C to 140°C by differential scanning calorimetry (DSC).

9. The crystal according to (1) or (2) characterized to have a characteristic absorption peak (2 θ degrees) at 12.4 (±0.2°) by XRD.

10. The crystal according to (1) or (2), characterized to have characteristic absorption peaks (2 θ degrees) at 11.1 (±0.2°), 11.3 (±0.2°), 12.2 (±0.2°), 12.4 (±0.2°), and 17.9 (±0.2°) by XRD.

11. The crystal according to (1) or (2), characterized to have characteristic absorption peaks (2 θ degrees) at 12.4 (±0.2°), 20.5 (±0.2°), 21.6 (±0.2°), 24.9 (±0.2°), 30.2 (±0.2°), 30.9 (±0.2°), 33.3 (±0.2°), and 37.8 (±0.2°) by XRD.

12. The crystal according to (1) or (2), characterized to have characteristic absorption peaks (2 θ degrees) at 11.1 (±0.2°), 11.3 (±0.2°), 12.2 (±0.2°), 12.4 (±0.2°), 17.9 (±0.2°), 20.5 (±0.2°), 21.6 (±0.2°), 22.6 (±0.2°), 24.9 (±0.2°), 29.1 (±0.2°), 30.2 (± 0.2°), 30.9 (±0.2°), 33.3 (±0.2°), and 37.8 (±0.2°) by XRD.

13. The crystal according to any one of (1), (2), and (10) to (12), characterized to have endothermic peaks at around 80°C, 143°C, and 150°C; have about 13.3 % weight decrease ratio at around 70°C; and have about 1.6 % weight decrease ratio at around 141°C by TG-DTA.

14. The crystal according to any one of (1), (2), and (10) to (13), characterized to have an endothermic peak at around 120°C to 140°C by DSC.

15. A pharmaceutical formulation which comprises, as an active ingredient, the crystal of 6-[4-(4-pyridylamino)phenyl]-4,5-dihydro-3(2H)-pyridazinone hydrochloride trihydrate according to any one of (1) to (14), and a pharmaceutically acceptable carrier.

16. A medicament for cardiac failure which comprises, as an active ingredient, the crystal of 6-[4-(4-pyridylamino)phenyl]-4,5-dihydro-3(2H)-pyridazinone hydrochloride trihydrate according to any one of (1) to (14), and a pharmaceutically acceptable carrier.

17. An antihypertensive agent which comprises, as an active ingredient, the crystal of 6-[4-(4-pyridylamino)phenyl]-4,5-dihydro-3(2H)-pyridazinone hydrochloride trihydrate according to any one of (1) to (14), and a pharmaceutically acceptable carrier.

18. An agent for enhancing calcium ion sensitivity which comprises, as an active ingredient, a crystal of 6-[4-(4-pyridylamino)phenyl]-4,5-dihydro-3(2H)-pyridazinone hydrochloride trihydrate according to any one of (1) to (14).

19. A cardiant which comprises, as an active ingredient, the crystal of 6-[4-(4-pyridylamino)phenyl]-4,5-dihydro-3(2H)-pyridazinone hydrochloride trihydrate according to any one of (1) to (14).

20. An ophthalmologic agent which comprises, as an active ingredient, the crystal of 6-[4-(4-pyridylamino)phenyl]-4,5-dihydro-3(2H)-pyridazinone hydrochloride trihydrate according to any one of (1) to (14).
